# EUROPÄISCHE PATENTSCHRIFT

(11) **EP 0 525 277 B1**
(45) Veröffentlichungstag und Bekanntmachung des Hinweises auf die Patenterteilung: **09.04.1997**
(21) Anmeldenummer: 92101316.5
(22) Anmeldetag: 28.01.1992
(51) Int. Cl.: B01L 3/14, G01N 21/11

(54) **Verfahren zum Einbringen von Reagenzien in eine Flüssigkeit sowie Reagenzieneinheit dafür**
Method for introduction of reagents into a liquid and test unit for it
Procédé pour introduire des agents chimiques dans un liquide et unité d'agents chimiques à ce but

(30) Priorität: 31.07.1991 DE 9109475 U
(43) Veröffentlichungstag der Anmeldung: 03.02.1993
(73) Patentinhaber: MACHEREY, NAGEL & CO., D-52355 Düren (DE)
(72) Erfinder: Radmacher, Dr., Edmund, W-5160 Düren (DE); Heering, Karl-Heinz, W-5166 Kreuzau (DE)
(74) Vertreter: Paul, Dieter-Alfred, Dipl.-Ing.

(56) Entgegenhaltungen:
- DE-A- 1 816 225
- DE-U- 8 703 659
- US-A- 4 125 376

## Beschreibung

Die Erfindung betrifft ein Verfahren zum Einbringen von Reagenzien in eine zu untersuchende Flüssigkeit innerhalb eines Reaktionsgefäßes, bei dem einerseits das Reagenz in getrocknetem Zustand in einem offenen Behälter gehalten und andererseits das Reaktionsgefäß zur Verfügung gestellt und dann für die Durchführung einer Analyse einander derart zugeführt werden, daß die in das Reaktionsgefäß eingefüllte Flüssigkeit in Kontakt mit der Reagenz kommt und dieses auflöst, sowie eine Reagenzieneinheit zum Einbringen von Reagenzien in eine zu untersuchende Flüssigkeit in Reaktionsgefäßen, mit einem offenen Behälter und einem darin gehaltenen, verfestigten, insbesondere gefriergetrockneten Reagenz.

Insbesondere bei der chemischen Wasseranalytik nimmt die photometrische Schnellanalyse einen festen Platz ein. Bei der Photometrie wird eine vorhandene oder zu erzeugende Färbung erfaßt und in Zusammenhang mit der Konzentration der zu messenden bzw. nachzuweisenden Substanz gebracht. Soweit die Substanz ihre Färbung nicht von Natur aus besitzt, wird der Wasserprobe ein geeignetes Reagenz - auch in Form einer Mischung - zugegeben, das eine gefärbte Verbindung entstehen läßt. Im Photometer wird das Licht vor der Messung zerlegt und nur der Teil des Spektrums verwendet, in dem die gefärbte Verbindung absorbiert. Das Maß der Absorption entspricht dann der Konzentration der nachzuweisenden Substanz.

Soweit die Reagenzien in gelöster Form nicht haltbar sind, werden sie in zunehmendem Maße als Lyophilisate, also in gefriergetrockneter Form, eingesetzt. Solche Lyophilisate zeichnen sich durch lange Haltbarkeit, sehr schnelle Löslichkeit, genaue Dosierungsmöglichkeit sowie einfache und sichere Handhabung aus. Im gattungsbildenden Stand der Technik wird das Lyophilisat in einem als lösbare Verschlußkappe ausgebildeten Behälter zur Verfügung gestellt (vgl. DE-U-87 0̸3 659.2). Hierzu weist die Verschlußkappe innenseitig eine napfartige Vertiefung auf, in die hinein das Reagenz dosiert und in der es dann lyophilisiert wird. Die solchermaßen ausgestatteten Verschlußkappen passen auf Reaktionsgefäße wie Meßkolben oder Rundküvetten, in die die zu analysierende Wasserprobe eingefüllt wird. Durch Umschwenken des jeweiligen Reaktionsgefäßes kommt die Wasserprobe mit dem in der Verschlußkappe gehaltenen Lyophilisat in Berührung. Hierdurch löst sich das Lyophilisat in der Wasserprobe und führt zu der gewünschten Reaktion.

Die Zuführung des Lyophilisats zur Wasserprobe mit Hilfe einer auf das Reaktionsgefäß aufzusetzenden Verschlußkappe ist mit einer Reihe von Nachteilen verbunden. So muß die Verschlußkappe für jede weitere Reagenzzugabe durch eine andere Verschlußkappe ersetzt werden. Dabei kann der Anwender mit Flüssigkeitsresten der Wasser- bzw. Analysenprobe in Kontakt kommen und hierdurch gefährdet werden, denn die Analysenprobe kann vor allem nach Reagenzzugabe unangenehme oder sogar gefährliche Eigenschaften haben, z. B. eine stark saure oder alkalische Lösung darstellen oder starke Färbungen bis hin zu toxischen Wirkungen aufweisen. Hinzu kommt, daß der in der Verschlußkappe verbliebene Rest der Analysenprobe zu einer Beeinträchtigung der Analysenpräzision führt. Außerdem ist nicht gesichert, weil von der Handhabung des Reaktionsgefäßes abhängig, daß das Lyophilisat vollständig aufgelöst wird. Auch dies beeinträchtigt die Analysenpräzision.

Daneben ist es aus der US-A-4 125 376 bekannt, eine vorgefertigte Einheit aus Reaktionsgefäß mit einem darin enthaltenen Schaumstoffwürfel, der mit einer Reagenzlösung imprägniert ist, bereitzustellen. Eine solche Einheit ist allenfalls zur Vornahme von qualitativen Analysen geeignet, weil nicht sicher feststellbar ist, welches Reagenzvolumen von dem Schaumwürfel aufgenommen worden ist und wieviel davon bei der Durchführung der Analyse herausgelöst wird. Weitere Nachteile bestehen darin, daß sich das Reagenz nur langsam aus dem Schaumstoffwürfel herauslösen kann und daß eine mehrstufige Analyse nicht möglich ist. Zudem besteht die Gefahr, daß sich Schaumstoffragmente von dem Schaumstoffwürfel lösen und bei der photometrischen Schnellanalyse stören. Hinzu kommt ein hoher Raumbedarf, wenn man für alle Analysenfälle gerüstet sein will. Entsprechend hoch ist auch das Entsorgungsvolumen.

Schließlich ist es auch seit nahezu 20̸ Jahren bekannt, sogenannte Indikatortabletten zu verwenden, bei denen das Reagenz in gebundener Form ohne Umhüllung bereitgestellt wird. Mit solchen Tabletten ist jedoch keine hohe Dosiergenauigkeit erzielbar. Außerdem werden mit der Tablette weitere Substanzen, nämlich Binde- und Sprengmittel, in die zu analysierende Flüssigkeit eingebracht. Außerdem lösen sich solche Tabletten nur sehr langsam auf.

Der Erfindung liegt deshalb die Aufgabe zugrunde, ein Verfahren zum Einbringen von Reagenzien in eine zu untersuchende Flüssigkeit so zu gestalten, daß das Einbringen des Reagenzes sicherer und einfacher sowie ohne Beeinträchtigung der Analysenpräzision durchgeführt werden kann. Die Aufgabe besteht ferner in der Bereitstellung einer Reagenzieneinheit, die besonders für die Durchführung des Verfahrens geeignet ist.

Diese Aufgabe wird - was das Verfahren angeht - dadurch gelöst, daß die Zuführung des Behälters mit dem darin gehaltenen Reagenz und des Reaktionsgefäßes durch Einwerfen des Behälters in das Reaktionsgefäß geschieht. Bei dieser Einbringung des Reagenzes in die zu untersuchende Flüssigkeit ist unabhängig davon, ob das Einwerfen bei schon im Reaktionsgefäß befindlicher Flüssigkeit oder schon vorher in das leere Reaktionsgefäß erfolgt und die Flüssigkeit erst dann eingefüllt wird, gesichert, daß das Reagenz vollständig aufgelöst wird, was eine hohe Analysenpräzision zur Folge hat. Außerdem ist die Gefahr, daß der Anwender über den offenen Behälter mit der Analysenprobe in Berührung kommt, wesentlich herabgesetzt. Dies gilt insbesondere dann, wenn nach Bedarf mehrere Kapseln gleichzeitig oder nacheinander in das Reaktionsgefäß eingeworfen werden, um die Konzentration des Reagenzes zu erhöhen.

Zweckmäßig ist es, daß für den bzw. die Behälter ein Material verwendet wird, das spezifisch schwerer oder leichter als die zu untersuchende Flüssigkeit ist. Dies hat zur Folge, daß der bzw. die eingeworfenen Behälter entweder auf den Boden des Reaktionsgefäßes absinken oder nach oben auf der Flüssigkeit aufschwimmen und somit nicht den Strahlengang des Photometers stören. Die Entsorgung des oder der Behälter erfolgt dann zusammen mit dem Reaktionsgefäß.

Ein für die Durchführung des erfindungsgemäßen Verfahrens besonders geeignete Reagenzieneinheit ist dadurch gekennzeichnet, daß der Behälter lediglich als eine das Reagenz einhüllende, offene Kapsel ausgebildet ist und keine Mittel für einen lösbaren Verschluß eines Reaktionsgefäßes aufweist, wobei die Kapsel selbstverständlich größenmäßig auf die bekannten Reaktionsgefäße so abgestimmt ist, daß sie durch den Hals des Reaktionsgefäßes paßt. Sie soll also nicht - wie im Stand der Technik - als auf das Reaktionsgefäß aufschraubbare Verschlußkappe ausgebildet sein, sondern entsprechend dem erfindungsgemäßen Verfahren auf die reine Kapselform reduziert sein, beispielsweise indem sie nur aus einer Zylinderwandung und einer Bodenwandung besteht.

Innenseitig sollte Kapsel Vorsprünge z.B. in Form von einem oder mehreren Ringstegen oder von Noppen haben, um den Halt des Lyophilisats in der Kapsel zu verbessern.

Aus den schon oben genannten Gründen ist es ferner zweckmäßig, daß die Kapsel aus einem Material besteht, das spezifisch schwerer oder leichter als Wasser ist, damit die Kapsel nach dem Einwerfen auf den Boden des Reaktionsgefäßes absinkt oder auf der Analysenprobe aufschwimmt. Es versteht sich, daß hierfür insbesondere Kunststoffe in Frage kommen, die chemisch inaktiv sind.

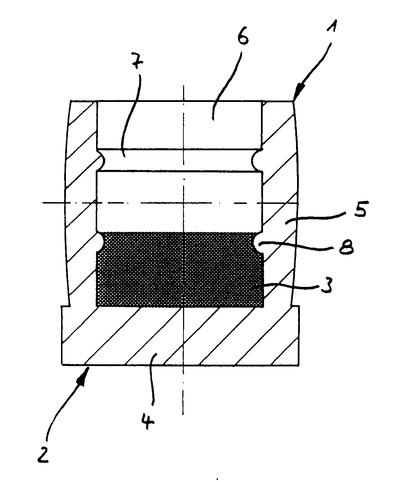

In der Zeichnung ist die Erfindung an Hand eines Ausführungsbeispiels näher veranschaulicht. Sie zeigt eine Reagenzieneinheit (1) im Längsschnitt und in stark vergrößerter Darstellung.

Die Reagenzieneinheit (1) besteht im wesentlichen aus einer Kapsel (2) und einem darin enthaltenen Lyophilisat (3), das das Reagenz bildet. Die Kapsel (2) weist eine kreisrunde Bodenwandung (4) von 7 mm Durchmesser auf, von der eine Ringzylinderwandung (5) hochsteht. Obenseitig ist die Kapsel (2) offen.

Das Lyophilisat (3) ist in dem von der Ringzylinderwandung (5) umschlossenen Innenraum (6) angeordnet und steht von der Bodenwandung (4) hoch. Die Füllhöhe kann entsprechend der gewünschten Dosierung verschieden gewählt werden. Um das Lyophilisat (3) besser zu halten, stehen von der Innenseite der Ringzylinderwandung (5) zwei im Abstand übereinander angeordnete Ringstege (7, 8) vor. Im dargestellten Beispiel hat nur der untere Ringsteg Berührung mit dem Lyophilisat (3).

Als Material für die Kapsel (2) kommen geeignete Kunststoffe, die für die vorgesehenen Analysen chemisch inaktiv und zudem in Wasser schwimmfähig sind, in Frage.

## Patentansprüche

1. Verfahren zum Einbringen eines Reagenzes (3) in eine zu untersuchende Flüssigkeit innerhalb eines Reaktionsgefäßes, bei dem einerseits das Reagenz (3) in getrocknetem Zustand in einem offenen Behälter (2) gehalten und andererseits das Reaktionsgefäß zur Verfügung gestellt und dann für die Durchführung einer Analyse einander derart zugeführt werden, daß die in das Reaktionsgefäß eingefüllte Flüssigkeit in Kontakt mit dem Reagenz (3) kommt und dieses auflöst,
dadurch gekennzeichnet, daß die Zuführung des Behälters (2) mit dem darin gehaltenen Reagenz (3) und des Reaktionsgefäßes durch Einwerfen des Behälters (2) in das Reaktionsgefäß geschieht.

2. Verfahren nach Anspruch 1,
dadurch gekennzeichnet, daß zur Untersuchung derselben Flüssigkeit mehrere Behälter (2) in das Reaktionsgefäß eingeworfen werden.

3. Verfahren nach Anspruch 2,
dadurch gekennzeichnet, daß die Behälter (2) nacheinander eingeworfen werden.

4. Verfahren nach einem der Ansprüche 1 bis 3,
dadurch gekennzeichnet, daß für den bzw. die Behälter (2) ein Material verwendet wird, das spezifisch schwerer oder leichter als die zu untersuchende Flüssigkeit ist.

5. Reagenzieneinheit zum Einbringen von Reagenzien in eine zu untersuchende Flüssigkeit in Reaktionsgefäßen, mit einem offenen Behälter und einem darin gehaltenen, verfestigten, insbesondere gefriergetrockneten Reagenz,
dadurch gekennzeichnet, daß der Behälter lediglich als eine das Reagenz (3) einhüllende, offene Kapsel (2) ausgebildet ist und keine Mittel für einen lösbaren Verschluß eines Reaktionsgefäßes aufweist.

6. Reagenzieneinheit nach Anspruch 5,
dadurch gekennzeichnet, daß die Kapsel (2) aus einer Zylinderwandung (5) und einer Bodenwandung (4) besteht.

7. Reagenzieneinheit nach Anspruch 5 oder 6,
dadurch gekennzeichnet, daß die Kapsel (2) innenseitig Vorsprünge (7, 8) aufweist.

8. Reagenzieneinheit nach Anspruch 7,
dadurch gekennzeichnet, daß die Vorsprünge aus einem oder mehreren Ringstegen (7, 8) bestehen.

9. Reagenzieneinheit nach einem der Ansprüche 5 bis 8,
dadurch gekennzeichnet, daß die Kapsel (2) aus einem Material besteht, das spezifisch schwerer oder leichter als Wasser ist.

## Claims

1. A method of introducing a reagent (3) into a liquid to be tested inside a reaction vessel, in which firstly the reagent (3) is held in a dry state in an open container (2) and secondly the reaction vessel is made available and they are then introduced to each other for performing an analysis in such a way that the liquid introduced into the reaction vessel comes into contact with the reagent (3) and dissolves the latter, characterised in that the introduction of the container (2) with the reagent (3) held therein and of the reaction vessel is effected by inserting the container (2) into the reaction vessel.

2. A method according to claim 1,
characterised in that a plurality of containers (2) is inserted into the reaction vessel for testing the same liquid.

3. A method according to claim 2,
characterised in that the containers (2) are inserted in succession.

4. A method according to any one of claims 1 to 3,
characterised in that a material which has a higher or lower specific gravity than that of the liquid to be tested is used for the container or containers (2).

5. A test unit for introducing reagents into a liquid to be tested in reaction vessels, having an open container and a consolidated reagent, particularly a freeze-dried reagent, contained therein,
characterised in that the container is constructed simply as an open capsule (2) which envelopes the reagent (3) and comprises no means for a soluble seal of a reaction vessel.

6. A test unit according to claim 5,
characterised in that the capsule (2) consists of a cylinder wall (5) and a bottom wall (4).

7. A test unit according to claim 5 or 6,
characterised in that the capsule (2) has projections (7, 8) on the inside.

8. A test unit according to claim 7,
characterised in that the projections consist of one or more annular ribs (7, 8).

9. A test unit according to any one of claims 5 to 8,
characterised in that the capsule (2) consists of a material which has a higher or lower specific gravity than that of water.

## Revendications

1. Procédé pour introduire un réactif (3) dans un liquide à analyser à l'intérieur d'un réservoir de réaction, selon lequel, d'une part, on maintient le réactif (3) à l'état sec dans un récipient ouvert (2) et, d'autre part, on met à disposition le réservoir de réaction, à la suite de quoi, afin d'effectuer une analyse, on les rapproche l'un de l'autre de telle sorte que le liquide rempli dans le réservoir de réaction entre en contact avec le réactif (3) et dissout ce dernier, **caractérisé** en ce que le rapprochement du récipient (2), dans lequel est maintenu le réactif (3), et du réservoir de réaction s'effectue en introduisant le récipient (2) dans le réservoir de réaction.

2. Procédé selon la revendication 1, **caractérisé** en ce que plusieurs récipients (2) sont introduits dans le réservoir de réaction pour l'analyse du même liquide.

3. Procédé selon la revendication 2, **caractérisé** en ce que les récipients (2) sont introduits les uns à la suite des autres.

4. Procédé selon l'une des revendications 1 à 3, **caractérisé** en ce qu'on utilise pour le ou les récipients (2) un matériau qui est spécifiquement plus lourd ou plus léger que le liquide à analyser.

5. Unité de réactif pour introduire des réactifs dans un liquide à analyser dans des réservoirs de réaction, avec un récipient ouvert et un réactif solidifié, notamment lyophilisé, maintenu dans ce récipient, **caractérisée** en ce que le récipient est réalisé simplement sous la forme d'une capsule ouverte (2) enrobant le réactif (3), et ne présente pas de moyens pour une fermeture amovible d'un réservoir de réaction.

6. Unité de réactif selon la revendication 5, **caractérisée** en ce que la capsule (2) est constituée d'une paroi cylindrique (5) et d'une paroi de fond (4).

7. Unité de réactif selon la revendication 5 ou 6, **caractérisée** en ce que la capsule (2) présente des saillies (7, 8) sur le côté intérieur.

8. Unité de réactif selon la revendication 7, **caractérisée** en ce que les saillies consistent en une ou plusieurs nervures annulaires (7, 8).

9. Unité de réactif selon l'une des revendications 5 à 8, **caractérisée** en ce que la capsule (2) est réalisée en un matériau qui est spécifiquement plus lourd ou plus léger que l'eau.
